# EUROPÄISCHE PATENTANMELDUNG

(11) **EP 0 703 227 A1**
(43) Veröffentlichungstag der Anmeldung: **27.03.1996**
(21) Anmeldenummer: 95810565.2
(22) Anmeldetag: 12.09.1995
(51) Int. Cl.: C07D 251/52, C07D 251/46, D06P 1/642, D06M 13/358, C08K 5/3492

(54) **Wasserlösliche Antioxidantien, Verfahren zu deren Herstellung und deren Verwendung zur photochemischen und thermischen Stabilisierung von Polyamid-Fasermaterialien**

(30) Priorität: 19.09.1994 CH 2851/94
(71) Anmelder: CIBA-GEIGY AG, CH-4002 Basel (CH)
(72) Erfinder: Fuso, Francesco, Dr., CH-4106 Therwil (CH); Reinert, Gerhard, Dr., CH-4123 Allschwil (CH)

(57) **Zusammenfassung**

Die vorliegende Erfindung betrifft neue wasserlösliche Antioxidantien der Formel 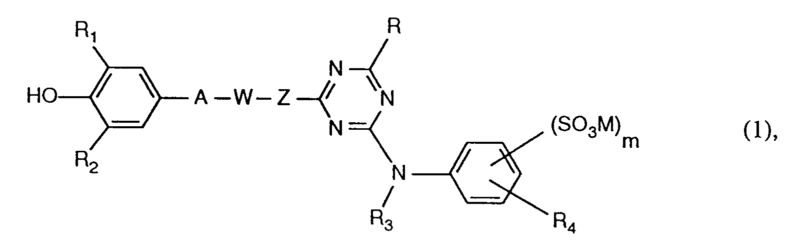
worin die Variablen die in den Ansprüchen angegebenen Bedeutungen haben,

Verfahren zu ihrer Herstellung und ihre Verwendung zur photochemischen und thermischen Stabilisierung von Polyamid-Fasermaterialien und -Färbungen.

## Beschreibung

Die vorliegende Erfindung betrifft neue wasserlösliche Antioxidantien, Verfahren zu deren Herstellung und ihre Verwendung zur photochemischen und thermischen Stabilisierung von Polyamid-Fasermaterialien und -Färbungen.

Es ist bereits aus der EP-A-0 466 647 oder EP-A-0 459 950 bekannt, Polyamidfasern photochemisch und thermisch zu stabilisieren, indem man sie mit bestimmten Verbindungen aus der Klasse der sterisch gehinderten Amine, sogenannten "HALS"-Stabilisatoren, behandelt. Es hat sich jedoch gezeigt, dass die damit erreichte Stabilisierung höchsten Ansprüchen nicht immer vollauf genügt. Es besteht daher Bedarf nach Verbindungen, die einen verbesserten Schutz der Polyamid-Fasermaterialien gegen Licht und Wärmeeinwirkung bieten.
Die Aufgabe der vorliegenden Erfindung war daher die Herstellung neuer Verbindungen, welche diese Ansprüche erfüllen.

Es wurde nun gefunden, dass man mit den nachfolgend beschriebenen speziellen Anti-oxidantien eine bessere Stabilisierung von Polyamidfasermaterialien erreichen kann.

Die neuen wasserlöslichen Antioxidantien zeichnen sich durch eine besonders gute Affinität zur Faser und einen guten Ausziehgrad, vor allem im neutralen pH-Bereich, aus.

Gegenstand der Erfindung sind somit neue wasserlösliche Antioxidantien der allgemeinen Formel 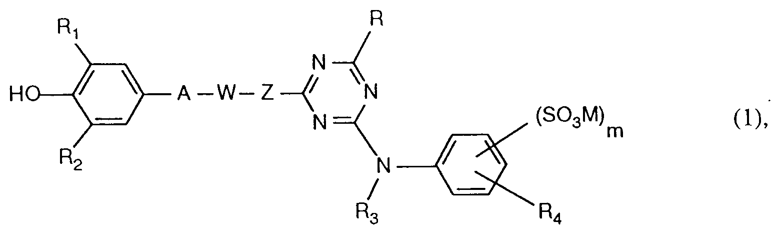
worin
R Halogen; C₁-C₅-Alkyl; Phenyl-C₁-C₅-alkyl; C₁-C₅-Alkoxy, wobei ab C₂ die Kette im Alkylrest durch ein Sauerstoffatom oder ein Schwefelatom unterbrochen sein kann; C₃-C₅-Alkenyloxy; C₄-C₈-Cycloalkyloxy; Amino; Mono- oder Di(phenyl-C₁-C₅-alkyl)-amino; unsubstituiertes oder durch Hydroxy oder Carboxy substituiertes Mono- oder Di-C₁-C₅-alkylamino, wobei die Alkylkette durch ein Sauerstoffatom, durch ein Schwefelatom oder durch SO₂ unterbrochen sein kann; Mono- oder Di-C₃-C₅-alkenylamino; unsubstituiertes oder durch C₁-C₅-Alkyl substituiertes Mono- oder Di-C₄-C₈-cycloalkylamino; unsubstituiertes oder durch Halogen, C₁-C₅-Alkyl, C₁-C₅-alkoxycarbonyl, C₁-C₅-Alkoxy, Carboxy, Carbamoyl, Mono- oder Di-C₁-C₅-alkanoylamino oder C₁-C₅-Alkanoyl substituiertes Phenoxy; Phenyl; Phenylthio; Phenyl-C₁-C₅-alkylthio; C₁-C₅-Alkylthio; C₄-C₈-Cycloalkylthio; unsubstituiertes oder durch C₁-C₅-Alkyl, Hydroxy oder Carboxy substituiertes 1-Azacycloalkyl; unsubstituiertes oder durch C₁-C₅-Alkyl substituiertes Morpholino; einen Rest der Formel 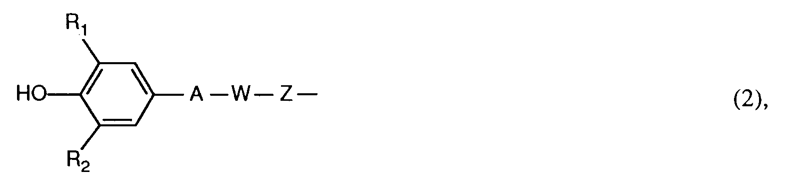
einen Rest der Formel 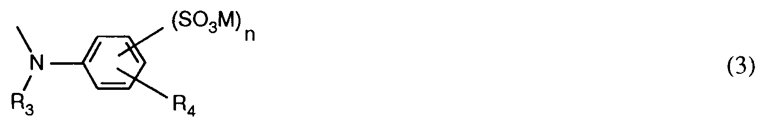
oder einen Rest der Formel 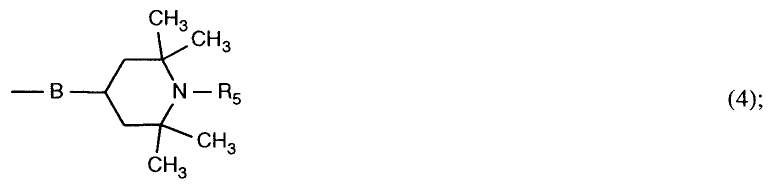
worin
einer der beiden Substituenten R₁ und R₂ Wasserstoff; C₁-C₈-Alkyl; C₅-C₇-Cycloalkyl; Phenyl-C₁-C₄-alkyl oder Phenyl; und der andere der beiden Substituenten R₁ und R₂ C₁-C₈-Alkyl; C₅-C₇-Cycloalkyl; Phenyl-C₁-C₄-alkyl oder Phenyl;
R₃ Wasserstoff oder C₁-C₄-Alkyl;
R₄ Wasserstoff; Halogen; Hydroxy; C₁-C₅-Alkyl; C₁-C₅-alkoxycarbonyl; C₁-C₅-Alkoxy; C₁-C₅-Alkanoyl; Carboxy; Amino; Mono- oder Di-C₁-C₅-alkylamino; oder Mono- oder Di-C₁-C₅-alkanoylamino;
R₅ Wasserstoff; Oxyl; Hydroxy; C₁-C₅-Alkyl; C₂-C₅-Alkenyl; C₁-C₅-Alkoxy; C₁-C₅-Alkanoyl; Benzoyl oder Benzyl;
m die Zahl 0, 1 oder 2;
n die Zahl 0, 1 oder 2;
und die Summe m + n 1, 2, 3 oder 4;
M gleich oder verschieden sein können und Wasserstoff; Alkalimetall-, Erdalkalimetall- oder Ammonium-Kation oder ein organisches Ammonium-Kation der Formel

(C₁-C₄-Alkyl)ₐ(H)_{b}N⁺

bedeuten, worin
a die Zahl 0 bis 3;
b die Zahl 1 bis 4; und die Summe a + b = 4 ist;
A die direkte Bindung oder unsubstituiertes oder durch Phenyl substituiertes C₁-C₈-Alkylen, wobei ab C₂ die Alkylenkette durch ein Sauerstoffatom oder ein Schwefelatom unterbrochen sein kann, und ab C₃ die Alkylenkette gerade oder verzweigt sein kann;
B Sauerstoff oder einen Rest -N(R₆)-, worin R₆ Wasserstoff oder C₁-C₆-Alkyl bedeutet;
W die direkte Bindung; -O-CO-; -NH-CO-; -CO-NH-G-; oder -CO-O-G- ist, worin G die direkte Bindung, C₁-C₆-Alkylen, C₅-C₈-Cycloalkylen, Phenylen oder der Rest -CH₂-C₆H₄-CH₂- ist, und -CO-NH-G- mit Z einen Ring bilden kann;
und
Z Sauerstoff; Schwefel oder ein Rest -N(R₇)- , worin R₇ Wasserstoff, C₁-C₄-Alkyl, Allyl oder Benzyl bedeutet;
sind.

R als C₁-C₅-Alkylthio ist z.B. Methylthio, Ethylthio, Propylthio oder Butylthio.
R als C₄-C₈-Cycloalkyloxy ist z.B. Cyclobutyloxy, Cyclopentyloxy, Methylcyclohexyloxy, Ethylcyclohexyloxy, Cycloheptyloxy, Cyclooctyloxy oder bevorzugt Cyclohexyloxy.
R als Mono- oder Di-C₄-C₈-cycloalkylamino ist z.B. Monocyclohexylamino oder bevorzugt Dicyclohexylamino. R als C₄-C₈-Cycloalkylthio ist z.B. Cycloheptylthio oder bevorzugt Cyclohexylthio.
R als Mono- oder Di-C₃-C₅-alkenylamino ist zum Beispiel Monobutenylamino, Monoallylamino, Diallylamino oder Dibutenylamino. Bevorzugte Reste sind dabei Monoallylamino oder Diallylamino.
R als Phenyl-C₁-C₅-alkyl ist z.B. Phenethyl, Phenylpropyl, Phenylbutyl oder vorzugsweise Benzyl.
R als Mono- oder Di(Phenyl-C₁-C₅-alkyl)amino ist z.B. Monobenzylamino, Monophenethylamino, Dibenzylamino, Diphenethylamino oder Benzylphenethylamino.
R als Phenyl-C₁-C₅-alkylthio ist z.B. Benzylthio oder Phenethylthio.
R als 1-Azocycloalkyl ist z.B. 1-Pyrrolidyl oder Piperidino.
R als Phenoxy kann durch die genannten Substituenten ein- oder mehrfach substituiert sein.
R als C₃-C₅-Alkenyloxy ist z.B. Butenyloxy oder Allyloxy.

R und R₄ als Mono- und Di-C₁-C₅-alkylamino ist z.B. N-Methylamino, N,N-Dimethylamino, N-Ethylamino, N,N-Diethylamino, N,N-Dipropylamino oder N-Methyl-N-ethylamino.

R und R₄ als Halogen bedeutet z.B. Fluor, Brom und vorzugsweise Chlor.

R, R₄ und R₅ als C₁-C₅-Alkoxy ist z.B. Methoxy, Ethoxy, Isopropoxy, Isobutoxy, tert.Butoxy oder tert.Amyloxy.

R, R₄ und R₅ als C₁-C₅-Alkyl ist z.B. Methyl, Ethyl, n-Propyl, Isopropyl, n-Butyl, Isobutyl, sek.Butyl, tert.Butyl, Amyl oder Isoamyl.

R₁ und R₂ als C₁-C₈-Alkyl ist z.B. Methyl, Ethyl, n-Propyl, Isopropyl, n-Butyl, Isobutyl, sek.Butyl, tert.Butyl, Pentyl, Hexyl, Heptyl oder Octyl. Bevorzugt sind Methyl, Ethyl, n-Propyl, Isopropyl, n-Butyl, Isobutyl, sek.Butyl, tert.Butyl. Besonders bevorzugt sind Methyl und tert. Butyl.
R₁ und R₂ als Phenyl-C₁-C₄-alkyl ist z.B. Phenethyl, Phenylpropyl, Phenylbutyl oder vorzugsweise Benzyl.
R₁ und R₂ als C₅-C₇ Cycloalkyl ist z.B. Cyclopentyl, Cyclohexyl, Methylcyclohexyl oder Cyclopheptyl.

R₃ und R₇ als C₁-C₄-Alkyl ist z.B. Methyl, Ethyl, n-Propyl, Isopropyl, n-Butyl, Isobutyl, sek.Butyl oder tert.Butyl.

R₄ als Mono-oder Di-C₁-C₅-alkanoylamino ist z.B. Formyl-, Acetyl-, Propionyl-, Butyryl-, Diformyl-, Diacetyl-, Dipropionyl-, Dibutyryl- oder Formyl-acetyl-amino.

R₄ als C₁-C₅-alkoxycarbonyl ist z.B. Methoxycarbonyl, Ethoxycarbonyl, Propoxycarbonyl, Butoxycarbonyl oder Pentoxycarbonyl.

R₄ und R₅ als C₁-C₅-Alkanoyl ist z.B. Formyl, Acetyl, Propionyl oder n-Butyryl.

R₅ als C₂-C₅-Alkenyl ist z.B. Vinyl, Butenyl oder vorzugsweise Allyl.
R₅ als C₁-C₅-Alkanoyl ist z.B. Formyl, Acetyl, Propionyl oder n-Butyryl.
Unter R₅ als Oxyl ist ein an den Stickstoff gebundenes Sauerstoff-radikal zu verstehen.

R₆ als C₁-C₆-Alkyl ist z.B. Methyl, Ethyl, n-Propyl, Isopropyl, n-Butyl, Isobutyl, sek.Butyl, tert.Butyl, Pentyl oder Hexyl.

A als C₁-C₈-Alkylen ist z.B. Methylen, Ethylen, Propylen, Tetramethylen, Pentamethylen oder Hexamethylen.

G als C₁-C₆-Alkylen ist z.B. Methylen, Ethylen, Propylen, Tetramethylen, Pentamethylen oder Hexamethylen.
G als C₅-C₈-Cycloalkylen ist z.B. Cyclopentylen, Cyclohexylen, Cycloheptylen oder Cyclooctylen.

Als Beispiele für Alkalimetallionen M seien das Lithium-, Natrium- oder Kalium-Kation genannt. Bevorzugt ist das Natrium-Kation. Beispiele für Erdalkalimetallionen sind das Calcium- und das Magnesium-Kation.
Für M als Ammonium-Kation entsprechend der Formel (C₁-C₄-Alkyl)ₐ(H)_{b}N⁺ kommt Trimethylammonium oder vorzugsweise Triethylammonium in Betracht.

Bevorzugte Ausführungen der erfindungsgemässen Verbindungen betreffen Verbindungen der Formel (1), worin
a) R ein Rest der Formel 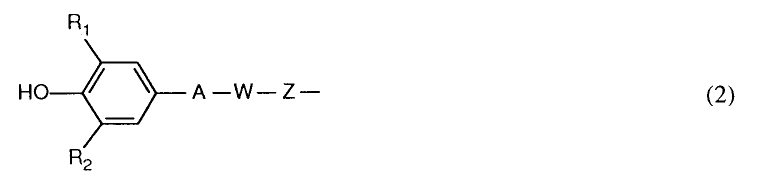 ist, worin
   einer der beiden Substituenten R₁ und R₂ Wasserstoff; C₁-C₈-Alkyl; C₅-C₇-Cycloalkyl; Phenyl-C₁-C₄-alkyl oder Phenyl, insbesondere C₁-C₄-Alkyl und vor allem Methyl oder tert.Butyl; und der andere der beiden Substituenten R₁ und R₂ C₁-C₈-Alkyl; C₅-C₇-Cycloalkyl; Phenyl-C₁-C₄-alkyl oder Phenyl, insbesondere C₁-C₄-Alkyl und vor allem Methyl oder tert.Butyl ist;
   A die direkte Bindung oder unsubstituiertes oder durch Phenyl substituiertes C₁-C₈-Alkylen ist, wobei ab C₂ die Alkylenkette durch ein Sauerstoffatom oder ein Schwefelatom unterbrochen sein kann, und ab C₃ die Alkylenkette gerade oder verzweigt sein kann;
   W die direkte Bindung; -O-CO-; -NH-CO-; -CO-NH-G-; oder -CO-O-G- ist, worin G die direkte Bindung, C₁-C₆-Alkylen, C₅-C₈-Cycloalkylen, Phenylen oder der Rest -CH₂-C₆H₄-CH₂- ist, und -CO-NH-G- mit Z einen Ring bilden kann;
   und
   Z Sauerstoff; Schwefel oder ein Rest -N(R₇)- ist, worin R₇ Wasserstoff, C₁-C₄-Alkyl, Allyl oder Benzyl bedeutet.
b) R ein Rest der Formel 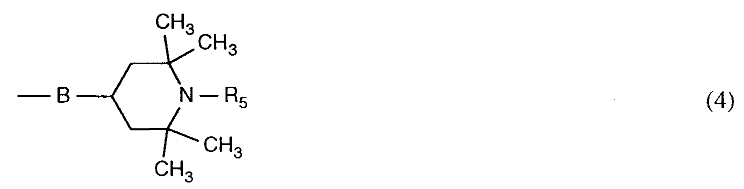 ist, worin
   R₅ Wasserstoff; Oxyl; Hydroxy; C₁-C₅-Alkyl; C₂-C₅-Alkenyl; C₁-C₅-Alkoxy; C₁-C₅-Alkanoyl; Benzoyl oder Benzyl; und
   B Sauerstoff oder ein Rest -N(R₆)-, worin R₆ Wasserstoff oder C₁-C₆-Alkyl bedeutet; sind.
c) R der Rest der Formel (4) ist und m = 2 ist.
d) A die direkte Bindung ist.
e) Z ein Rest -N(R₇)- ist, worin R₇ Wasserstoff, C₁-C₄-Alkyl, Allyl oder Benzyl bedeutet.
f) m = 1 ist.
g) R₃ Wasserstoff ist.
h) R₄ Wasserstoff oder C₁-C₅-Alkyl ist.
i) R₅ Wasserstoff oder C₁-C₅-Alkyl ist.

Besonders bevorzugt werden Verbindungen der Formel (1), worin A die direkte Bindung und Z ein Rest -N(R₇)- sind, worin R₇ Wasserstoff, C₁-C₄-Alkyl, Allyl oder Benzyl bedeutet.

Einen weiteren Gegenstand der Erfindung stellt das Verfahren zur Herstellung der neuen wasserlöslichen Antioxidantien der Formel (1) dar.

Die Herstellung der erfindungsgemässen wasserlöslichen Triazinverbindungen der Formel (1) erfolgt z.B. dadurch, dass man ein Mol einer 2,4,6-Trihalogen-s-triazinverbindung nacheinander mit einem oder zwei Mol der Verbindung der Formel 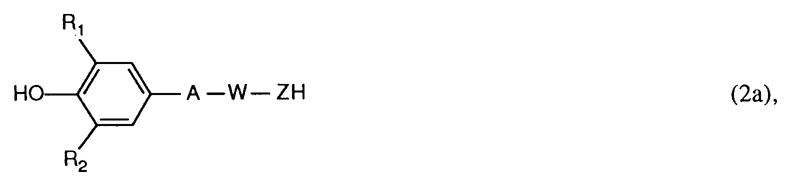
worin R₁, R₂, A, W und Z die in Formel (1) angegebene Bedeutung haben, sowie mit einem oder zwei Mol der Verbindung der Formel 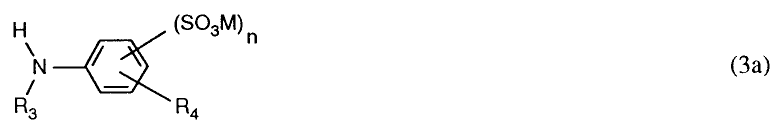
worin R₃, R₄, M und n die in Formel (1) angegebene Bedeutung haben, und, falls je eine Verbindung der Formel (2a) und (3a) verwendet werden, mit einer den Substituent R', einführenden Verbindung umsetzt, wobei die Reihenfolge der einzelnen Reaktionsschritte beliebig ist.
Substituent R' hat die in Formel (1) angegebene Bedeutung für R, ausgenommen die Reste der Formel (2) und (3).
In der Regel geht man so vor, dass man im ersten Reaktionsschritt die 2,4,6-Trihalogen-s-triazinverbindung mit der Verbindung umsetzt, die die geringere Reaktivität aufweist.

Die Reaktionstemperatur liegt zwischen 0 und 100°C, vorzugsweise zwischen 20 und 80°C, die Reaktionszeit zwischen 1 und 20, vorzugsweise zwischen 1 und 4 Stunden.

Die bei den Kondensationsreaktionen entstehende Halogenwasserstoffsäure kann durch das Endprodukt selbst oder durch Hinzufügen einer weiteren Base, wie beispielsweise wässrigem Ammoniak, Alkalimetallhydroxiden, Alkalimetallcarbonaten, -hydrogen-carbonaten oder einer organischen Base, wie beispielsweise Triethylamin, abgefangen werden. Vorzugsweise wird als Base Alkalimetallcarbonat, wie z.B. Natriumcarbonat, verwendet.

Die Umsetzungen erfolgen üblicherweise in wässriger Lösung gegebenenfalls mit Zusatz von organischen Lösungsmitteln. Die als Ausgangsverbindungen verwendeten 2,4,6-Trihalogen-s-triazinverbindungen sind allgemein bekannt. Sie werden dabei vorzugsweise als wässrige Suspensionen eingesetzt. Eine besonders bevorzugte Ausgangsverbindung ist Cyanurchlorid.
Die übrigen für die Herstellung der Verbindungen der Formel (1) verwendeten Ausgangsverbindungen wie z.B. die die Reste der Formeln (2), (3) oder (4) einführenden Verbindungen , wie z.B. Metanilsäure, Anilin-2,5-disulfonsäure, 2,6-Di-tert.butyl-4-aminophenol oder 6-(3'-Methyl-5'-tert.butyl-4'hydroxyphenyl)-hexanol sind ebenfalls allgemein bekannt.

Alle Verbindungen gemäss Formel (1) werden vorzugsweise als Natriumsalze hergestellt. Dazu werden sie beispielsweise mit der äquivalenten Menge Natronlauge gelöst und als Lösung, Dispersion oder Emulsion für eine Anwendung formuliert.

Die neuen erfindungsgemässen wasserlöslichen Antioxidantien gemäss Formel (1) eignen sich dazu, die thermische und photochemische Stabilität von ungefärbten und gefärbten Polyamid-Fasermaterialien zu erhöhen. Die Verwendung der erfindungsgemässen Verbindungen zur Erhöhung der thermischen und photochemischen Stabilität von Polyamidfasern und -färbungen stellt somit einen weiteren Gegenstand der vorliegenden Erfindung dar.

Die neuartigen Verbindungen der Formel (1) können in übliche Textilveredlungsprozesse für Polyamidfasern integriert werden.
Die Verbindungen der Formel (1 ) werden erfindungsgemäss aus wässrigem Bad appliziert, das die Verbindungen in einer Menge von z.B. 0,005 bis 10 Gew.-%, vorzugsweise 0,05 bis 2 Gew.-% enthält. Vorzugsweise werden die Verbindungen dem Färbebad zugefügt.
Die Applikation kann vor, während oder nach dem Färben, nach einem Auszieh- oder Kontinueverfahren erfolgen. Die Applikation während des Färbens ist bevorzugt.
Die Verbindungen der Formel (1) können auch in den üblichen Druckpasten beim Textildruck eingesetzt werden.

Beim Ausziehverfahren kann das Flottenverhältnis innerhalb eines weiten Bereiches gewählt werden, z.B. 1:3 bis 1:200, vorzugsweise 1:10 bis 1:40. Man arbeitet zweckmässig bei einer Temperatur von 20 bis 120°C, vorzugsweise 40 bis 100°C.

Beim Kontinueverfahren beträgt der Flottenauftrag zweckmässig 40-700, vorzugsweise 40-500 Gew.-%. Das Fasermaterial wird dann einem Hitzebehandlungsprozess unterworfen, um die applizierten Farbstoffe und die Antioxidantien zu fixieren. Dieses Fixieren kann auch nach der Kalt-Verweil-Methode erfolgen.

Die Hitzebehandlung der Kontinuefärbungen und Drucke erfolgt vorzugsweise durch ein Dämpfverfahren unter Behandlung in einem Dämpfer mit gegebenenfalls überhitztem Dampf bei einer Temperatur von 98 bis 105°C während z.B. 1-7, vorzugsweise 1-5 Minuten. Die Fixierung der Farbstoffe gemäss dem Kaltverweilverfahren kann durch Lagerung der imprägnierten und vorzugsweise aufgerollten Ware bei Raumtemperatur (15 bis 30°C) z.B. während 3 bis 24 Stunden erfolgen, wobei die Kaltverweilzeit bekanntlich vom Farbstoff abhängig ist.

Nach Beendigung des Färbeprozesses bzw. der Fixierung werden die hergestellten Färbungen auf übliche Weise gewaschen und getrocknet.

Man erhält nach der vorliegenden Erfindung ungefärbte und gefärbte Fasermaterialien mit guter thermischer und/oder photochemischer Stabilität.

Als die erfindungsgemäss zu stabilisierenden Färbungen kommen solche in Betracht, die durch Dispersions-, Säure- oder Metallkomplexfarbstoffe, besonders Azo-, 1:2-Metallkomplexfarbstoffe, z.B. 1:2-Chrom-, 1:2-Kobaltkomplexfarbstoffe oder Cu-Komplexfarbstoffe erzeugt werden.

Beispiele für solche Farbstoffe sind in Colour Index, 3. Auflage, 1971, Band 4, beschrieben.

Unter Polyamid-Fasermaterial wird synthetisches Polyamid, wie z.B. Polyamid-6, Polyamid-6,6 oder Polyamid-12, sowie modifiziertes Polyamid, z.B. basisch anfärbbares Polyamid verstanden. Neben den reinen Polyamidfasern kommen vor allem auch Fasermischungen aus Polypropylen und Polyamid, Polyamid/Wolle oder Polyurethan und Polyamid in Betracht, so z.B. Trikotmaterial aus Polyamid/Polyurethan im Mischungsverhältnis 70:30. Grundsätzlich kann das reine oder gemischte Polyamidmaterial in den verschiedensten Verarbeitungsformen vorliegen, wie z.B. als Faser, Garn, Gewebe, Gewirke, Vlies oder Flormaterial.

Vor allem Färbungen auf Polyamid-Fasermaterial,gegebenenfalls in Mischung mit Polyurethan oder Polypropylen, die Licht und/oder Hitze ausgesetzt werden und z.B. als Teppich, Badebekleidung oder Autopolsterstoff vorliegen, eignen sich besonders gut dazu, nach dem vorliegenden Verfahren behandelt zu werden

Das vorliegende Verfahren eignet sich besonders vorteilhaft zur Behandlung von Polyamidfasermaterial, das Licht und Hitze ausgesetzt wird und z.B. als Autopolsterstoff oder Teppich Verwendung findet.

Die folgenden Beispiele veranschaulichen die Erfindung. Teile bedeuten Gewichtsteile und Prozente Gewichtsprozente.

### Beispiel 1:

Eine neutralisierte Anschlämmung von 15,0 g eines Kondensationsproduktes von Cyanurchlorid mit zwei Aequivalenten Metanilsäure (Gehalt 84,5%) in 50 ml Wasser/10 ml Dioxan wird mit einer Lösung von 6,8 g 3,5-Di-tert.-butyl-4-hydroxy-benzylamin-hydrochlorid in 75 ml Dioxan/25 ml Wasser/12,5 ml 2M Natronlauge versetzt. Anschliessend erwärmt man unter Rühren und unter Stickstoff auf 60° C und hält gleichzeitig den pH-Wert des Reaktionsgemisches durch Zudosieren von 2M Natronlauge bei 8. Nach 24 Stunden kühlt man auf Raumtemperatur und filtriert von unlöslichen Anteilen ab. Das Filtrat wird mit 2M Salzsäure angesäuert bis pH 3,5 und bei 50° C mit 17,5 g Natriumchlorid versetzt. Man lässt das Reaktionsgemisch erkalten, filtriert den Niederschlag ab und wäscht mit einem Gemisch aus Dioxan/10%-iger wässriger Lösung von Natriumchlorid 30 Minuten nach.
Nach dem Trocknen bei 70° C im Vakuum erhält man 7,95 g eines beigefarbenen Pulvers. Dieses wird zur weiteren Reinigung nochmals in 65 ml 10%-iger wässriger Lösung von Natriumchlorid/15 ml Aethanol/1 g Natriumhydrogencarbonat heiss gelöst, mit 2M Salzsäure bis pH 4 angesäuert und auf Raumtemperatur abgekühlt. Der entstandene Niederschlag wird abgenutscht, mit 2%-iger wässriger Lösung von Natriumchlorid gewaschen und im Vakuum bei 70° C getrocknet Man erhält 3,9 g einer Verbindung der Formel

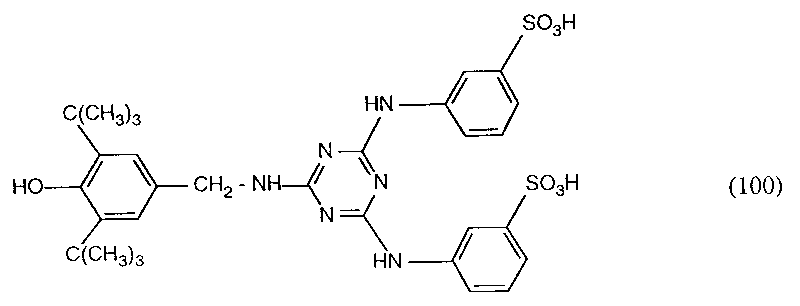

### Beispiel 2:

Eine Anschlämmung von 9,0 g eines Kondensationsproduktes (Gehalt 84,5 %) von Cyanurchlorid mit zwei Aequivalenten Metanilsäure in 100 ml Wasser/50 ml Dioxan wird mit 3,86 g 2,6-Di-tert-butyl-4-aminophenol Hydrochlorid versetzt. Man inertisiert mit Stickstoff und stellt durch Zudosieren von 2M Natronlauge den pH-Wert des Reaktionsgemisches auf 7,5. Anschliessend erwärmt man auf 62 - 64° C und rührt während 18 Stunden nach, wobei der pH-Wert durch Zudosieren von 2M Natronlauge bei 7,5 gehalten wird. Anschliessend wird das Reaktionsgemisch auf Raumtemperatur gekühlt und mit zwei Portionen je à 50 ml Essigsäure-ethylester extrahiert. Die wässrige Phase wird mit 2M Salzsäure bis pH 3 angesäuert und portionsweise mit 10%-iger wässriger Natriumchloridlösung versetzt. Der Niederschlag wird abgenutscht, nacheinander mit 50 ml einer 10%-igen und 25 ml einer 5%-igen wässrigen Natriumchloridlösung gewaschen und im Vakuum bei 70° C getrocknet. Man erhält 7,78 g einer Verbindung der Formel

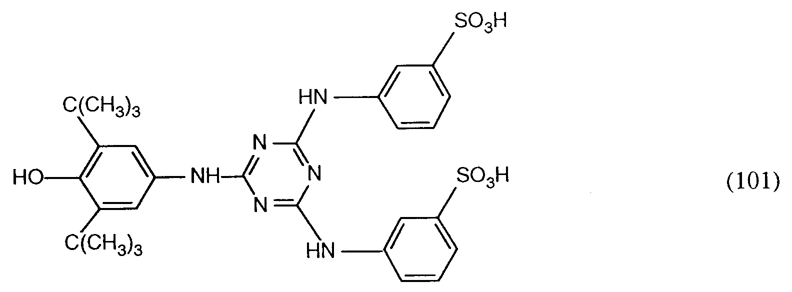

### Beispiel 3:

Eine Lösung von 8,67 g eines Monokondensationsproduktes (Gehalt 74%) aus Cyanurchlorid und Anilin-2,5-disulfonsäure in 100 ml Wasser wird mit 3,86 g 2,6-Di-tert-butyl-4-aminophenol Hydrochlorid und 50 ml Dioxan versetzt. Es wird mit Stickstoff inertisiert und durch Zudosieren von 2M Natronlauge schwach alkalisch (pH = 7,5) gestellt. Anschliessend erwärmt man während 19 Stunden auf 42 - 44° C und hält dabei den pH-Wert durch kontinuierliches Zudosieren von 2M Natronlauge weiterhin bei 7,5. Danach wird auf Raumtemperatur abgekühlt und das Reaktionsgemisch filtriert. Das Filtrat enthält das Produkt der Formel

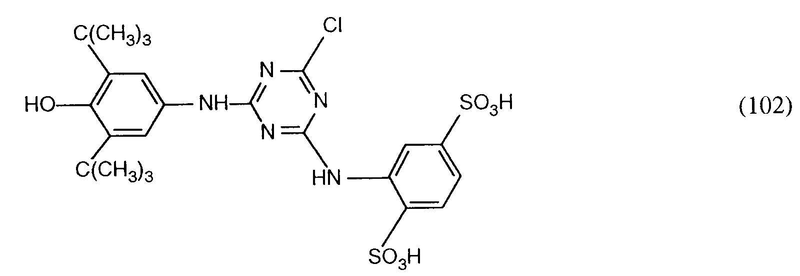

Das erhaltene Filtrat wird bei Raumtemperatur mit 2,34 g 4-Amino-2,2,6,6-tetramethylpiperidin versetzt, auf 82-85° C erhitzt und während 21 Stunden bei dieser Temperatur gehalten. Die Reaktionslösung wird am Rotationsverdampfer eingedampft und der Rückstand durch Zugabe von 4 ml 2M Natronlauge in Lösung gebracht. Man versetzt diese Lösung nacheinander mit 20 g Natriumchlorid und 2 ml Ethanol und säuert mit 2,5 ml 2M Salzsäure an. Man rührt während 15 Stunden aus und filtriert den kristallinen Niederschlag ab. Nach dem Waschen mit 10%-iger wässrigen Natriumchloridlösung und Trocknen im Vakuum bei 70° C verbleiben 8,3 g einer Verbindung der Formel

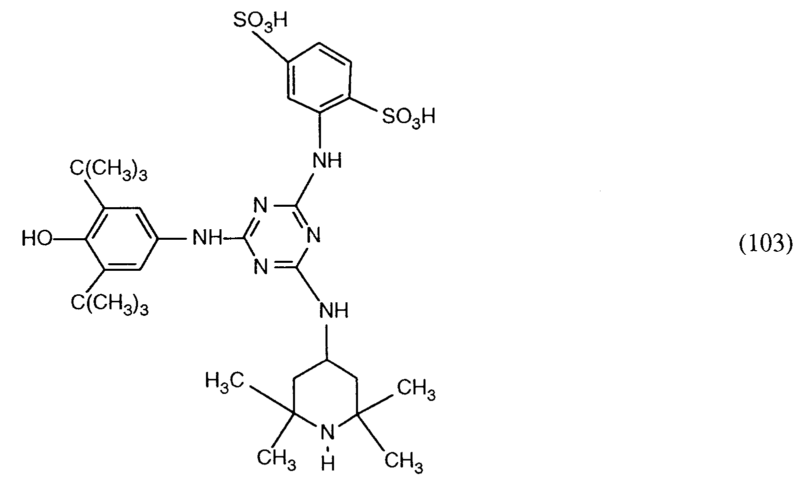

### Beispiel 4:

6,18 g eines Kondensationproduktes aus je einem Aequivalent 6-(3'-Methyl-5'-tert.butyl-4'-hydroxyphenyl)-hexanol und Cyanurchlorid werden in 40 ml Aceton gelöst. und unter Rühren bei Raumtemperatur in 10 ml Wassel eingetragen. Der pH-Wert wird durch Zutropfen von 2M Natronlauge auf 6,5-7 eingestellt.
Zu dieser Lösung wird 2,59 g Metanilsäure, gelöst in 30 ml 0,5M Natronlauge bei Raumtemperatur zugetropft und der pH-Wert durch Zugabe von 2M Natronlauge auf 7 gehalten. Anschliessend wird das Reaktionsgemisch auf 35° C aufgeheizt und bei dieser Temperatur 3 Stunden gehalten. Das Produkt wird durch Zugabe von 65 g konz. Natronlauge während 1 Stunde ausgesalzen. Anschliessend wird das Aceton unter Vakuum abdestilliert, die wässrige Dispersion abfiltriert und mit 10%-iger wässrigen Natriumchloridlösung gewaschen. Nach anschliessendem Trocknen bei 40° C im Vakuum erhält man eine Verbindung der Formel

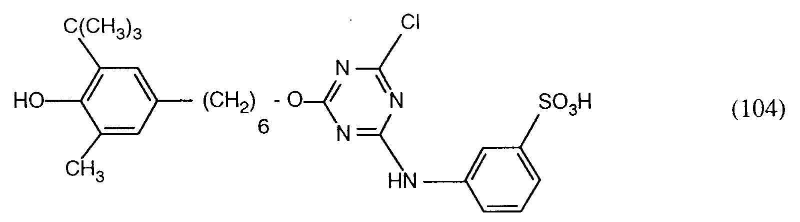

### Beispiel 5:

Verfährt man wie in Beispiel 4 beschrieben, setzt jedoch anstelle von einem Aequivalent Metanilsäure zwei Aequivalente Metanilsäure ein und erhöht die Reaktionstemperatur bis zum Rückfluss, erhält man eine Verbindung der Formel

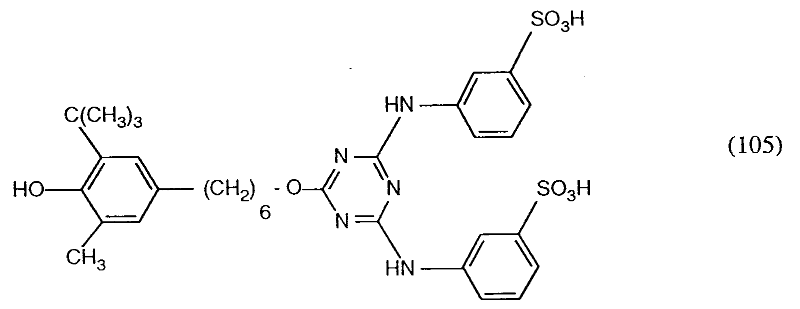

### Beispiel 6:

Verfährt man wie in Beispiel 4 beschrieben, setzt jedoch anstelle des Kondensationproduktes aus je einem Aequivalent 6-(3'-Methyl-5'-tert.butyl-4'-hydroxyphenyl)-hexanol und Cyanurchlorid die äquivalente Menge von 2,4-Di-chlor-6-(3',5'-di-tert.butyl-4'-hydroxy)phenoxy-s-triazin, erhält man eine Verbindung der Formel

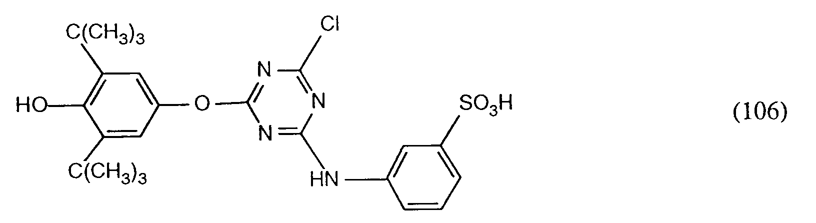

### Beispiel 7:

Verfährt man wie in Beispiel 4 beschrieben, setzt jedoch anstelle des Kondensationproduktes aus je einem Aequivalent 6-(3'-Methyl-5'-tert.butyl-4'-hydroxyphenyl)-hexanol und Cyanurchlorid die äquivalente Menge von 2,4-Di-chlor-6-(3',5'-di-tert.butyl-4'-hydroxy)phenoxy-s-triazin, sowie zwei Aequivalente Metanilsäure, erhält man eine Verbindung der Formel

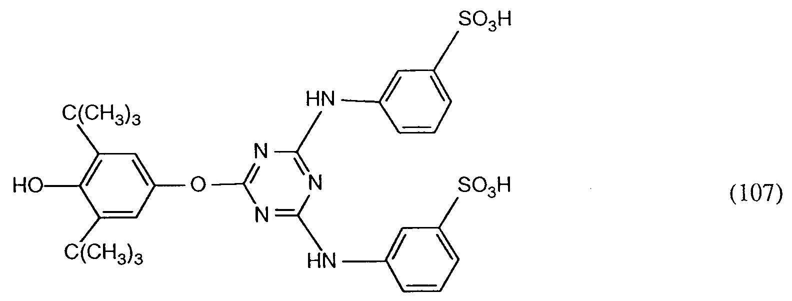

### Beispiel 8:

Eine Lösung von 0,1 mol Cyanurchlorid in 120 ml Aceton wird mit 3 g wasserfreies Natriumsulfat versetzt und auf 0° C gekühlt. Danach lässt man eine Lösung von 0,1 mol 3,5-Di-tert.butyl-4-hydroxy-thiophenol in 25 ml Chlorbenzol zulaufen. Anschliessend werden innerhalb von 20 Minuten bei 5° C 12,7 g s-Collidin zum Reaktionsmischung zugetropft. Zur Vervollständigung der Reaktion wird während 30 Minuten bei 5° C und 2 Stunden bei Raumtemperatur gerührt. Anschliessend wird das Reaktionsgemisch filtriert und mit 100 ml Aceton gewaschen. Das Filtrat wird in ein Gemisch von 400 g Eis und 900 ml Wasser eingerührt, mit verdünnter Salzsäure bis zur schwach lakmussauren Reaktion angesäuert und mit Essigester erschöpfend extrahiert. Nach der üblichen Aufarbeitung wird aus Hexan/Aceton umkristallisiert. Man erhält 16,9 g 2,4-Dichlor-6-(3',5'-di-tert.butyl-4'-hydroxyphenylsulfanyl)-s-triazin.
Diese Verbindung kann analog zum in Beispiel 4 beschriebenen Verfahren mit einem Aequivalent Metanilsäure umgesetzt werden. Man erhält eine Verbindung der Formel

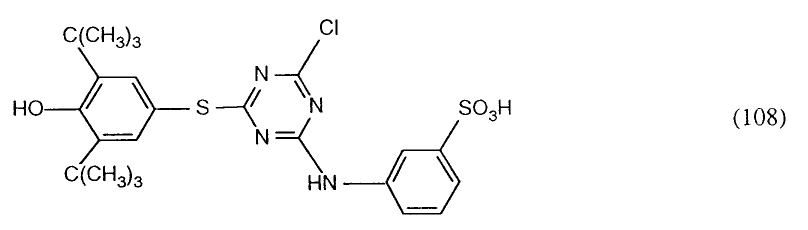

### Beispiel 9:

Verfährt man wie im Beispiel 8 beschrieben, setzt aber das Zwischenprodukt 2,4-Di-chlor-6-(3',5'-di-tert.butyl-4'-hydroxyphenylsulfanyl)- s-triazin analog zum in Beispiel 5 beschriebenen Verfahren mit zwei Aequivalenten Metanilsäure um, erhält man eine Verbindung der Formel

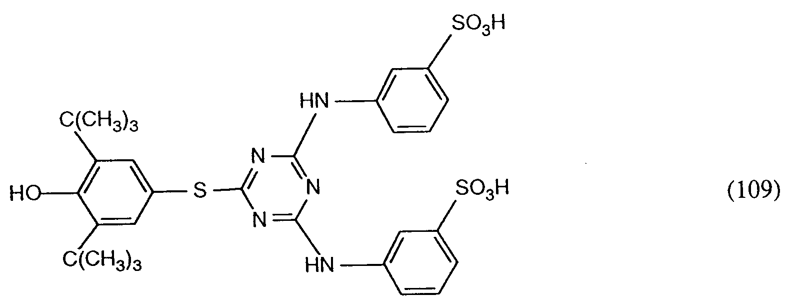

### Beispiel 10:

In einem ®AHIBA-Färbeapparat werden bei einem Flottenverhältnis von 1:30 drei Muster von je 10 g einer Polyamid-6-Maschenware behandelt. Die Flotten 1-3 enthalten jeweils folgende Zusätze: 0,5g/l Mono-Na-phosphat, 1,5 g/l Di-Na-phosphat und 1,0 Gew.-%, bezogen auf das Textilgut, eines handelsüblichen Egalisiermittels.
Flotte 2 enthält zusätzlich 1,0 Gew.-%, bezogen auf das Textilgut, der Verbindung der Formel (100).
Flotte 3 enthält zusätzlich 1,0 Gew.-%, bezogen auf das Textilgut, der Verbindung der Formel (101).

Die so vorbereiteten Flotten 1-3 werden auf 40°C erwärmt. Nach Zugabe des Textilmaterials behandelt man 10 Minuten bei dieser Temperatur und erwärmt während 30 Minuten auf 95°C. Nach einer Behandlungszeit von 10 Minuten bei 95°C werden jeder Flotte 2 Gew.-%, bezogen auf das Textilgut, 80 %iger Essigsäure zugesetzt und weitere 35 Minuten behandelt. Danach kühlt man auf 70°C ab, spült die Muster mit kaltem Wasser, zentrifugiert und trocknet sie bei 80° C.

Die Muster 1-3 werden anschliessend auf ihre photochemische und thermische Stabilität geprüft.
a) Zur Prüfung der photochemischen Stabilität werden aus den behandelten Muster 1 bis 3 12 x 8 cm grosse Stücke ausgeschnitten, 180 Stunden nach Deutscher Norm DIN 75.202 belichtet und gemäss der Schweizerischen Norm SN 198.461 auf ihre Reissfestigkeit und Dehnung geprüft.
b) Zur Prüfung der thermischen Beständigkeit werden die behandelten Muster 1 bis 3 in einem Ofen bei 150° C 96 Stunden gehalten und anschliessend werden gemäss der Schweizerischen Norm SN 198.461 die Reissfestigkeit und Dehnung, und gemäss der Deutschen Norm DIN 6167 die Vergilbung geprüft.

Die mit den Verbindungen der Formeln (100) und (101) behandelten Muster 2 und 3 zeigen dabei eine bessere Reissfestigkeit und Dehnung und eine geringere Vergilbung als das unbehandelte Muster 1.

### Beispiel 11:

In einem ®AHIBA-Färbeapparat werden bei einem Flottenverhältnis von 1:30 drei Muster von je 10 g einer Polyamid-6-Maschenware behandelt. Die Flotten 1a-3a enthalten jeweils folgende Zusätze: 0,5g/l Mono-Na-phosphat, 1,5 g/l Di-Na-phosphat und 1,0 Gew.-%, bezogen auf das Färbegut, eines handelsüblichen Egalisiermittels, 0,032 Gew.-% Farbstoff der Formel (I), 0,005 Gew.-% Farbstoff der Formel (II) und 0,002 %, jeweils bezogen auf das zu färbende Material, Farbstoff der Formel (III) in gelöster Form: 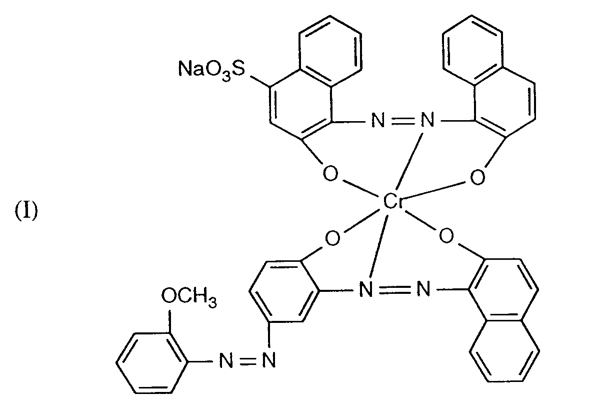 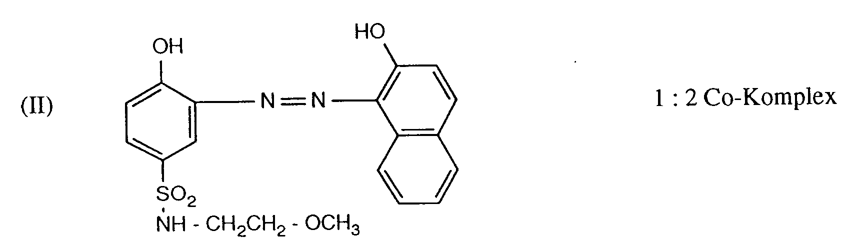
und 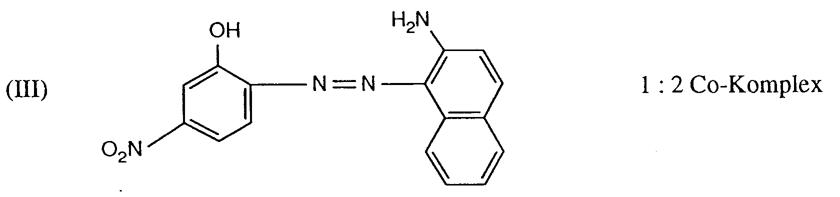
und 0,003 Gew.-%, bezogen auf das Färbegut, eines handelsüblichen Tensids.
Flotte 2a enthält zusätzlich 1,0 Gew.-%, bezogen auf das Färbegut, der Verbindung der Formel (100).
Flotte 3a enthält zusätzlich 1,0 Gew.-%, bezogen auf das Färbegut, der Verbindung der Formel (101).

Die Färbeflotten werden auf 45°C erwärmt. Nach Zugabe des Textilmaterials behandelt man 10 Minuten bei dieser Temperatur und erwärmt während 30 Minuten auf 95°C. Nach einer Färbezeit von 10 Minuten bei 95°C werden der Färbeflotten 2 Gew.-%, bezogen auf das Färbegut, 80 %iger Essigsäure zugesetzt und weitere 35 Minuten gefärbt. Danach kühlt man auf 70°C ab, spült das gefärbte Material mit kaltem Wasser und trocknet es bei 80° C.

Anschliessend wird die Lichtechtheit der Muster 1a-3a nach zwei verschiedenen Normen, a) Deutscher Norm DIN 75.202 (FAKRA) und b) USA-Norm SAE J 1885, geprüft.

Die mit den Verbindungen der Formeln (100) und (101) behandelten Muster 2a und 3a zeigen dabei eine bessere Lichtechtheit als das unbehandelte Muster 1a.

### Beispiel 12:

In einem ®AHIBA-Färbeapparat werden bei einem Flottenverhältnis von 1:30 drei Muster von je 10 g einer Polyamid-6-Maschenware behandelt. Die Flotten 1b-3b enthalten jeweils folgende Zusätze: 0,5g/l Mono-Na-phosphat, 1,5 g/l Di-Na-phosphat und 1,0 Gew.-%, bezogen auf das Textilgut, eines handelsüblichen Egalisiermittels.
Flotte 2b enthält zusätzlich 0,5 Gew.-%, bezogen auf das Textilgut, der Verbindung der Formel (103).
Flotte 3b enthält zusätzlich 1,0 Gew.-%, bezogen auf das Textilgut, der Verbindung der Formel (103).

Die so vorbereiteten Flotten 1b-3b werden auf 40°C erwärmt, das Fasermaterial zugegeben und wie im Beispiel 10 beschrieben, behandelt.

Die Muster 1b-3b werden anschliessend auf ihre photochemische und thermische Stabilität geprüft.
a) Zur Prüfung der photochemischen Stabilität werden aus den behandelten Muster 1b bis 3b 12 x 8 cm grosse Stücke ausgeschnitten, 180 Stunden nach Deutscher Norm DIN 75.202 belichtet und gemäss der Schweizerischen Norm SN 198.461 auf ihre Reissfestigkeit und Dehnung geprüft.
b) Zur Prüfung der thermischen Beständigkeit werden die behandelten Muster 1 bis 3 in einem Ofen bei 150° C 96 Stunden gehalten und anschliessend werden gemäss der Schweizerischen Norm SN 198.461 die Reissfestigkeit und Dehnung und gemäss der Deutschen Norm DIN 6167 die Vergilbung geprüft.

Die mit der Verbindung der Formel (103) behandelten Muster 2b und 3b zeigen dabei eine bessere photochemische und thermische Stabilität als das unbehandelte Muster 1b.

### Beispiel 13:

In einem ®AHIBA-Färbeapparat werden bei einem Flottenverhältnis von 1:30 drei Muster von je 10 g einer Polyamid-6-Maschenware behandelt. Die Flotten 1c-3c enthalten jeweils folgende Zusätze: 0,5g/l Mono-Na-phosphat, 1,5 g/l Di-Na-phosphat und 1,0 Gew.-%, bezogen auf das Färbegut, eines handelsüblichen Egalisiermittels, 0,032 Gew.-% Farbstoff der Formel (I) und 0,005 %, jeweils bezogen auf das zu färbende Material, Farbstoff der Formel (III) in gelöster Form.
Flotte 2c enthält zusätzlich 0,5 Gew.-%, bezogen auf das Färbegut, der Verbindung der Formel (103).
Flotte 3c enthält zusätzlich 1,0 Gew.-%, bezogen auf das Färbegut, der Verbindung der Formel (103).

Die Färbeflotten werden auf 45°C erwärmt, das Textilmaterial zugegeben und wie im Beispiel 11 beschrieben, gefärbt.
Anschliessend wird die Lichtechtheit der Muster 1c-3c nach zwei verschiedenen Normen, a) Deutscher Norm DIN 75.202 (FAKRA) und b) USA-Norm SAE J 1885, geprüft.

Die mit der Verbindung der Formel (103) behandelten Muster 2c und 3c zeigen dabei eine bessere Lichtechtheit als das unbehandelte Muster 1c.

### Beispiel 14:

In einem ®AHIBA-Färbeapparat werden bei einem Flottenverhältnis von 1:30 sieben Muster von je 10 g einer Polyamid-6-Maschenware behandelt. Die Flotten 1d-7d enthalten jeweils folgende Zusätze: 0,5g/l Mono-Na-phosphat, 1,5 g/l Di-Na-phosphat und 1,0 Gew.-%, bezogen auf das Textilgut, eines handelsüblichen Egalisiermittels.
Flotte 2d enthält zusätzlich 0,5 Gew.-%, bezogen auf das Textilgut, der Verbindung der Formel (104).
Flotte 3d enthält zusätzlich 0,5 Gew.-%, bezogen auf das Textilgut, der Verbindung der Formel (105).
Flotte 4d enthält zusätzlich 0,5 Gew.-%, bezogen auf das Textilgut, der Verbindung der Formel (106).
Flotte 5d enthält zusätzlich 0,5 Gew.-%, bezogen auf das Textilgut, der Verbindung der Formel (107).
Flotte 6d enthält zusätzlich 0,5 Gew.-%, bezogen auf das Textilgut, der Verbindung der Formel (108).
Flotte 7d enthält zusätzlich 0,5 Gew.-%, bezogen auf das Textilgut, der Verbindung der Formel (109).
Die so vorbereiteten Flotten ld-7d werden auf 40°C erwärmt. Nach Zugabe des Textilmaterials wird wie im Beispiel 10 beschrieben, behandelt.

Die Muster 1d bis 7d werden anschliessend auf ihre photochemische und thermische Stabilität geprüft.
a) Zur Prüfung der photochemischen Stabilität werden aus den behandelten Muster 1d-7d 12 x 8 cm grosse Stücke ausgeschnitten, 180 Stunden nach Deutscher Norm DIN 75.202 belichtet und gemäss der Schweizerischen Norm SN 198.461 auf ihre Reissfestigkeit und Dehnung geprüft.
b) Zur Prüfung der thermischen Beständigkeit werden die behandelten Muster 1d-7d in einem Ofen bei 150° C 96 Stunden gehalten und anschliessend werden gemäss der Schweizerischen Norm SN 198.461 die Reissfestigkeit und Dehnung und gemäss der Deutschen Norm DIN 6167 die Vergilbung geprüft.

Die mit den Verbindungen der Formeln (104) bis (109) behandelten Muster 2d bis 7d zeigen dabei eine bessere photochemische und thermische Stabilität als das unbehandelte Muster 1d.

## Patentansprüche

1. Wasserlösliche Antioxidantien der Formel 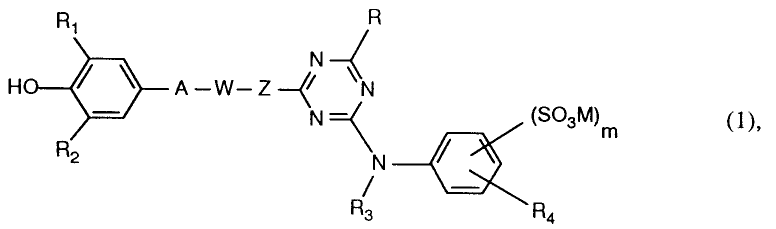 worin
R Halogen; C₁-C₅-Alkyl; Phenyl-C₁-C₅-alkyl; C₁-C₅-Alkoxy, wobei ab C₂ die Kette im Alkylrest durch ein Sauerstoffatom oder ein Schwefelatom unterbrochen sein kann; C₃-C₅-Alkenyloxy; C₄-C₈-Cycloalkyloxy; Amino; Mono- oder Diphenyl-C₁-C₅-alkylamino; unsubstituiertes oder durch Hydroxy oder Carboxy substituiertes Mono- oder Di-C₁-C₅-alkylamino, wobei die Alkylkette durch ein Sauerstoffatom, durch ein Schwefelatom oder durch SO₂ unterbrochen sein kann; Mono- oder Di-C₃-C₅-alkenylamino; unsubstituiertes oder durch C₁-C₅-Alkyl substituiertes Mono- oder Di-C₄-C₈-cycloalkylamino; unsubstituiertes oder durch Halogen, C₁-C₅-Alkyl, C₁-C₅-Alkoxycarbonyl, C₁-C₅-Alkoxy, Carboxy, Carbamoyl, Mono- oder Di-C₁-C₅-alkanoylamino oder C₁-C₅-Alkanoyl substituiertes Phenoxy; Phenyl; Phenylthio; Phenyl-C₁-C₅-Alkylthio; C₁-C₅-Alkylthio; C₄-C₈-Cycloalkylthio; unsubstituiertes oder durch C₁-C₅-Alkyl, Hydroxy oder Carboxy substituiertes 1-Azacycloalkyl; unsubstituiertes oder durch C₁-C₅-Alkyl substituiertes Morpholino; einen Rest der Formel 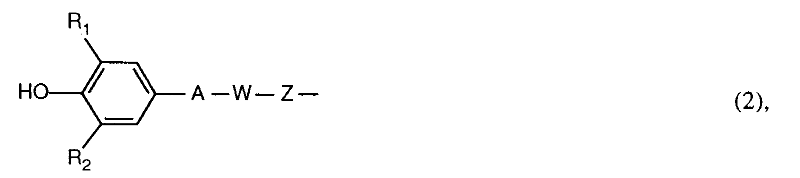 einen Rest der Formel 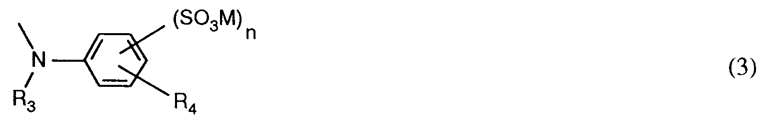 oder einen Rest der Formel 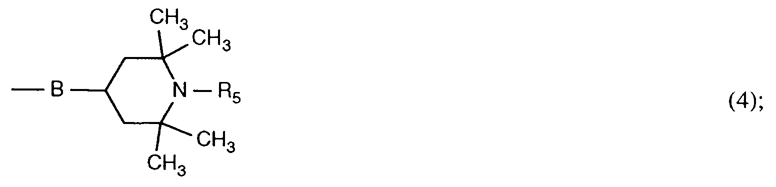 worin
ein der beiden Substituenten R₁ und R₂ Wasserstoff; C₁-C₈-Alkyl; C₅-C₇-Cycloalkyl; Phenyl-C₁-C₄-alkyl oder Phenyl; und das andere der beiden Substituenten R₁ und R₂ C₁-C₈-Alkyl; C₅-C₇-Cycloalkyl; Phenyl-C₁-C₄-alkyl oder Phenyl;
R₃ Wasserstoff oder C₁-C₄-Alkyl;
R₄ Wasserstoff; Halogen; Hydroxy; C₁-C₅-Alkyl; C₁-C₅-Alkoxycarbonyl; C₁-C₅-Alkoxy; C₁-C₅-Alkanoyl; Carboxy; Amino; Mono- oder Di-C₁-C₅-alkylamino; oder Mono- oder Di-C₁-C₅-alkanoylamino;
R₅ Wasserstoff; Oxyl; Hydroxy; C₁-C₅-Alkyl; C₂-C₅-Alkenyl; C₁-C₅-Alkoxy;
C₁-C₅-Alkanoyl; Benzoyl oder Benzyl;
m die Zahl 0, 1 oder 2;
n die Zahl 0, 1 oder 2;
und die Summe m + n 1, 2, 3 oder 4;
M gleich oder verschieden sein können und Wasserstoff; Alkalimetall-, Erdalkalimetalloder Ammonium-Kation oder ein organisches Ammonium-Kation der Formel
(C₁-C₄-Alkyl)ₐ(H)_{b}N⁺
bedeuten, worin
a die Zahl 0 bis 3;
b die Zahl 1 bis 4; und die Summe a + b = 4 ist;
A die direkte Bindung oder unsubstituiertes oder durch Phenyl substituiertes C₁-C₈-Alkylen, wobei ab C₂ die Alkylenkette durch ein Sauerstoffatom oder ein Schwefelatom unterbrochen sein kann, und ab C₃ die Alkylenkette gerade oder verzweigt sein kann;
B Sauerstoff oder ein Rest -N(R₆)-, worin R₆ Wasserstoff oder C₁-C₆-Alkyl bedeutet;
W die direkte Bindung; -O-CO-; -NH-CO-; -CO-NH-G-; oder -CO-O-G- ist, worin G die direkte Bindung, C₁-C₆-Alkylen, C₅-C₈-Cycloalkylen, Phenylen oder der Rest -CH₂-C₆H₄-CH₂- ist, und -CO-NH-G- mit Z einen Ring bilden kann;
und
Z Sauerstoff; Schwefel oder ein Rest -N(R₇)- , worin R₇ Wasserstoff, C₁-C₄-Alkyl, Allyl oder Benzyl bedeutet;
sind.

2. Wasserlösliche Antioxidantien gemäss Anspruch 1, dadurch gekennzeichnet, dass R ein Rest der Formel 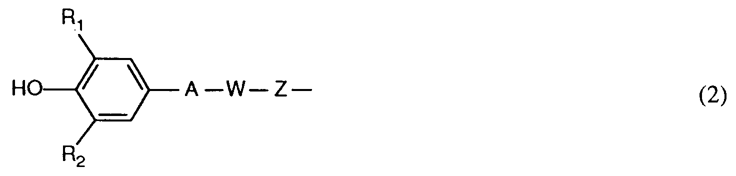 ist, worin
ein der beiden Substituenten R₁ und R₂ Wasserstoff; C₁-C₈-Alkyl; C₅-C₇-Cycloalkyl; Phenyl-C₁-C₄-alkyl oder Phenyl; und das andere der beiden Substituenten R₁ und R₂ C₁-C₈-Alkyl; C₅-C₇-Cycloalkyl; Phenyl-C₁-C₄-alkyl oder Phenyl ist;
A die direkte Bindung oder unsubstituiertes oder durch Phenyl substituiertes C₁-C₈-Alkylen ist, wobei ab C₂ die Alkylenkette durch ein Sauerstoffatom oder ein Schwefelatom unterbrochen sein kann, und ab C₃ die Alkylenkette gerade oder verzweigt sein kann;
W die direkte Bindung; -O-CO-; -NH-CO-; -CO-NH-G-; oder -CO-O-G- ist, worin G die direkte Bindung, C₁-C₆-Alkylen, C₅-C₈-Cycloalkylen, Phenylen oder der Rest -CH₂-C₆H₄-CH₂- ist, und -CO-NH-G- mit Z einen Ring bilden kann;
und
Z Sauerstoff; Schwefel oder ein Rest -N(R₇)- ist, worin R₇ Wasserstoff, C₁-C₄-Alkyl, Allyl oder Benzyl bedeutet.

3. Wasserlösliche Antioxidantien gemäss Anspruch 2, dadurch gekennzeichnet, dass R₁ und R₂ unabhängig voneinander C₁-C₄-Alkyl sind.

4. Wasserlösliche Antioxidantien gemäss Anspruch 3, dadurch gekennzeichnet, dass R₁ und R₂ unabhängig voneinander Methyl oder tert.Butyl sind.

5. Wasserlösliche Antioxidantien gemäss einem der Ansprüche 1 bis 4, dadurch gekennzeichnet, dass m = 1 ist.

6. Wasserlösliche Antioxidantien gemäss Anspruch 1, dadurch gekennzeichnet, dass R ein Rest der Formel 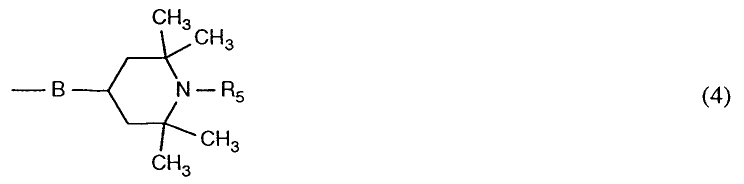 ist, worin
R₅ Wasserstoff; Oxyl; Hydroxy; C₁-C₅-Alkyl; C₂-C₅-Alkenyl; C₁-C₅-Alkoxy; C₁-C₅-Alkanoyl; Benzoyl oder Benzyl; und
B Sauerstoff oder ein Rest -N(R₆)-, worin R₆ Wasserstoff oder C₁-C₆-Alkyl bedeutet; sind.

7. Wasserlösliche Antioxidantien gemäss Anspruch 6, dadurch gekennzeichnet, dass m = 2 ist.

8. Wasserlösliche Antioxidantien gemäss einem der Ansprüche 1 bis 7, dadurch gekennzeichnet, dass A die direkte Bindung ist.

9. Wasserlösliche Antioxidantien gemäss einem der Ansprüche 1 bis 8, dadurch gekennzeichnet, dass Z ein Rest -N(R₇)- ist, worin R₇ Wasserstoff, C₁-C₄-Alkyl, Allyl oder Benzyl bedeutet.

10. Wasserlösliche Antioxidantien gemäss einem der Ansprüche 1 bis 7 dadurch gekennzeichnet, dass A die direkte Bindung und Z ein Rest -N(R₇)- sind, worin R₇ Wasserstoff, C₁-C₄-Alkyl, Allyl oder Benzyl bedeutet.

11. Wasserlösliche Antioxidantien gemäss einem der Ansprüche 1 bis 10, dadurch gekennzeichnet, dass R₃ Wasserstoff ist.

12. Wasserlösliche Antioxidantien gemäss einem der Ansprüche 1 bis 11, dadurch gekennzeichnet, dass R₄ Wasserstoff oder C₁-C₅-Alkyl ist.

13. Wasserlösliche Antioxidantien gemäss einem der Ansprüche 1 bis 13, dadurch gekennzeichnet, dass R₅ Wasserstoff oder C₁-C₅-Alkyl ist.

14. Wasserlösliche Antioxidantien gemäss Anspruch 1, dadurch gekennzeichnet, dass R Chlor; Rest der Formel (3), worin R₃ und R₄ Wasserstoff und n = 1 ist; oder Rest der Formel (4), worin R₅ Wasserstoff und B -NH- ist;
R₁ -C(CH₃)₃;
R₂ -CH₃; oder -C(CH₃)₃;
m die Zahl 1 oder 2;
M Natriumkation;
A die direkte Bindung; -CH₂; oder -(CH₂)₆;
W die direkte Bindung; -O-CO-; -NH-CO-; -CO-NH-G-; oder -CO-O-G- ist, worin G die direkte Bindung, C₁-C₆-Alkylen, C₅-C₈-Cycloalkylen, Phenylen oder der Rest -CH₂-C₆H₄-CH₂- ist, und -CO-NH-G- mit Z einen Ring bilden kann;
und
Z Sauerstoff; Schwefel oder -NH-
sind.

15. Verfahren zur Herstellung von wasserlöslichen Antioxidantien der Formel 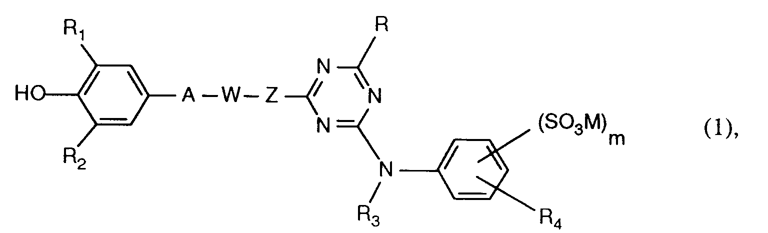 worin
R Halogen; C₁-C₅-Alkyl; Phenyl-C₁-C₅-alkyl; C₁-C₅-Alkoxy, wobei ab C₂ die Kette im Alkylrest durch ein Sauerstoffatom oder ein Schwefelatom unterbrochen sein kann; C₃-C₅-Alkenyloxy; C₄-C₈-Cycloalkyloxy; Amino; Mono- oder Diphenyl-C₁-C₅-alkylamino; unsubstituiertes oder durch Hydroxy oder Carboxy substituiertes Mono- oder Di-C₁-C₅-alkylamino, wobei die Alkylkette durch ein Sauerstoffatom, durch ein Schwefelatom oder durch SO₂ unterbrochen sein kann; Mono- oder Di-C₃-C₅-alkenylamino; unsubstituiertes oder durch C₁-C₅-Alkyl substituiertes Mono- oder Di-C₄-C₈-cycloalkylamino; unsubstituiertes oder durch Halogen, C₁-C₅-Alkyl, C₁-C₅-Alkoxycarbonyl, C₁-C₅-Alkoxy, Carboxy, Carbamoyl, Mono- oder Di-C₁-C₅-alkanoylamino oder C₁-C₅-Alkanoyl substituiertes Phenoxy; Phenyl; Phenylthio; Phenyl-C₁-C₅-Alkylthio; C₁-C₅-Alkylthio; C₄-C₈-Cycloalkylthio; unsubstituiertes oder durch C₁-C₅-Alkyl, Hydroxy oder Carboxy substituiertes 1-Azacycloalkyl; unsubstituiertes oder durch C₁-C₅-Alkyl substituiertes Morpholino; einen Rest der Formel 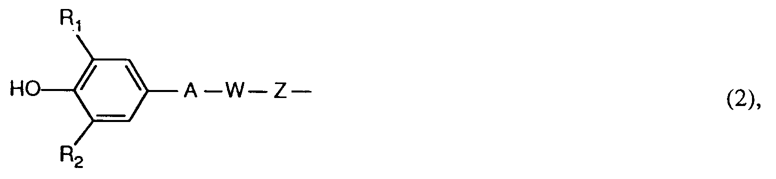 einen Rest der Formel 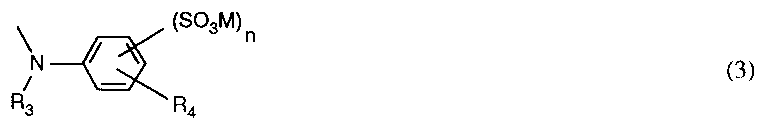 oder einen Rest der Formel 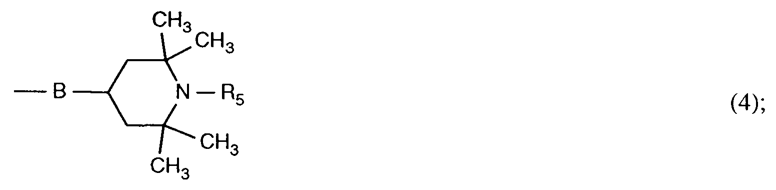 worin
einer der beiden Substituenten R1 und R₂ Wasserstoff; C₁-C₈-Alkyl; C₅-C₇-Cycloalkyl; Phenyl-C₁-C₄-alkyl oder Phenyl; und der andere der beiden Substituenten R₁ und R₂ C₁-C₈-Alkyl; C₅-C₇-Cycloalkyl; Phenyl-C₁-C₄-alkyl oder Phenyl;
R₃ Wasserstoff oder C₁-C₄-Alkyl;
R₄ Wasserstoff; Halogen; Hydroxy; C₁-C₅-Alkyl; C₁-C₅-Alkoxycarbonyl; C₁-C₅-Alkoxy; C₁-C₅-Alkanoyl; Carboxy; Amino; Mono- oder Di-C₁-C₅-alkylamino; oder Mono- oder Di-C₁-C₅-alkanoylamino;
R₅ Wasserstoff; Oxyl; Hydroxy; C₁-C₅-Alkyl; C₂-C₅-Alkenyl; C₁-C₅-Alkoxy; C₁-C₅-Alkanoyl; Benzoyl oder Benzyl;
m die Zahl 0, 1 oder 2;
n die Zahl 0, 1 oder 2;
und die Summe m + n 1, 2, 3 oder 4;
M gleich oder verschieden sein können und Wasserstoff; Alkalimetall-, Erdalkalimetall- oder Ammonium-Kation oder ein organisches Ammonium-Kation der Formel
(C₁-C₄-Alkyl)ₐ(H)_{b}N⁺
bedeuten, worin
a die Zahl 0 bis 3;
b die Zahl 1 bis 4; und die Summe a + b = 4 ist;
A die direkte Bindung oder unsubstituiertes oder durch Phenyl substituiertes C₁-C₈-Alkylen, wobei ab C₂ die Alkylenkette durch ein Sauerstoffatom oder ein Schwefelatom unterbrochen sein kann, und ab C₃ die Alkylenkette gerade oder verzweigt sein kann;
B Sauerstoff oder ein Rest -N(R₆)- , worin R₆ Wasserstoff oder C₁-C₆-Alkyl bedeutet;
W die direkte Bindung; -O-CO-; -NH-CO-; -CO-NH-G-; oder -CO-O-G- ist, worin G die direkte Bindung, C₁-C₆-Alkylen, C₅-C₈-Cycloalkylen, Phenylen oder der Rest -CH₂-C₆H₄-CH₂- ist, und -CO-NH-G- mit Z einen Ring bilden kann;
und
Z Sauerstoff; Schwefel oder ein Rest -N(R₇)- , worin R₇ Wasserstoff, C₁-C₄-Alkyl, Allyl oder Benzyl bedeutet;
sind, dadurch gekennzeichnet, dass man ein Mol einer 2,4,6-Trihalogen-s-triazinverbindung nacheinander mit einem oder zwei Mol der Verbindung der Formel 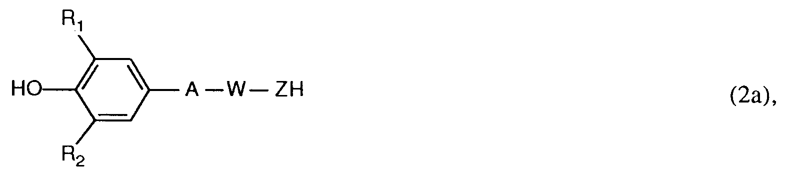 worin R₁, R₂, A, W und Z die in Formel (1) angegebene Bedeutung haben, so wie mit einem oder zwei Mol der Verbindung der Formel 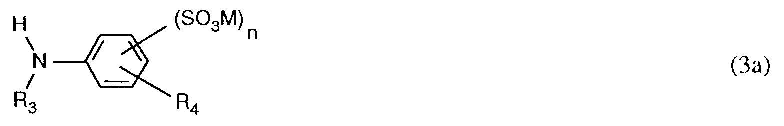 worin R₃, R₄, M und n die in Formel (1) angegebene Bedeutung haben, und, falls je eine Verbindung der Formel (2a) und (3a) verwendet werden, gegebenenfalls mit einer den Substituenten R' einführenden Verbindung, worin R' die in Formel (1) angegebene Bedeutung für R, ausgenommen die Reste der Formel (2) und (3) hat, umsetzt.

16. Verfahren zur photochemischen und thermischen Stabilisierung von Polyamidfasermaterialien, dadurch gekennzeichnet, dass man gefärbte oder ungefärbte Polyamid-Fasermaterialien mit wasserlöslichen Antioxidantien der Formel (1) gemäss Anspruch 1 behandelt.

17. Verwendung der wasserlöslichen Antioxidantien gemäss einem der Ansprüche 1 bis 14 zur photochemischen und thermischen Stabilisierung von Polyamid-Fasermaterialien und -Färbungen.

18. Das mit den wasserlöslichen Antioxidantien gemäss einem der Ansprüche 1 bis 14 behandelte Fasermaterial.
